# FASCICULE DE BREVET EUROPEEN

(11) **EP 0 654 303 B1**
(45) Date de publication et mention de la délivrance du brevet: **28.01.1998**
(21) Numéro de dépôt: 94402458.7
(22) Date de dépôt: 31.10.1994
(51) Int. Cl.: B04B 5/04, A61M 1/02

(54) **Procédé de traitement par centrifugation d'un liquide conditionné dans des poches à parois souples, reliées à au moins un filtre**
Verfahren zur Zentrifugalbehandlung einer Flüssigkeit, eingefüllt in Taschen mit flexiblen Wänden, verbunden mit wenigstens einem Filter
Method for centrifugal treatment of a liquid packed in flexible bags, connected with at least one filter

(30) Priorité: 23.11.1993 FR 9314003
(43) Date de publication de la demande: 24.05.1995
(73) Titulaire: JOUAN, Société Anonyme dite, F-44600 Saint Nazaire (FR)
(72) Inventeur: Lambert, Roland Jean Luc Raoul, F-44400 Reze (FR); Letourneur, Jean-Claude Charles Michel, F-44380 Pornichet (FR)
(74) Mandataire: Bernasconi, Jean

(56) Documents cités:
- EP-A- 0 499 891
- US-A- 4 439 177
- US-A- 5 100 564

## Description

La présente invention concerne la centrifugation de liquide en vue de la préparation de produits tels que des composés sanguins, préparation qui nécessite, après centrifugation du liquide, un filtrage du produit centrifugé afin d'en séparer les composants.

L'invention concerne, plus particulièrement, la centrifugation d'ensembles formés par au moins une poche à parois souples reliée à un filtre par l'intermédiaire d'une tubulure souple.

Dans les procédés de préparation de produits sanguins, le produit de départ, le sang, est transféré, puis centrifugé, et enfin filtré pour en séparer les composants, toutes ces opérations devant être effectuées dans des conditions de stérilité rigoureuses. Le produit de départ est donc, avant centrifugation, conditionné dans des poches à parois souples reliées entre elles par des tubulures souples, mais comportant parfois dans le circuit un filtre. Ce dernier est généralement constitué d'une enveloppe rigide de forme plate, dont le volume intérieur est séparé en deux parties par une paroi filtrante.

Les filtres et les poches devant rester reliés pour éviter tout risque de contamination, on n'a donc pas d'autre choix que de placer dans une enceinte de centrifugeuse l'ensemble formé par les poches et les filtres qui leur sont reliés (voir US-A-5100564 ou EP-A-499891).

Cette opération de centrifugation de poches et de filtres dans une même enceinte, c'est-à-dire dans un même espace de confinement, comporte les difficultés suivantes:

Les filtres étant de forme plate et comportant un volume interne ne contenant que du gaz et une paroi filtrante, ils sont vulnérables non seulement vis-à-vis des forces centrifuges développées sur leurs parois, mais également vis-à-vis des pressions exercées par les poches voisines sur leurs parois, au cours de la centrifugation.

La pression régnant dans le liquide contenu dans les poches soumises à la centrifugation, atteint de façon typique plusieurs kilogrammes, voire plusieurs dizaines de kilogrammes par centimètre carré, ce qui rend les poches elles-mêmes extrêmement fragiles vis-à-vis de l'action que pourraient avoir sur elles des corps solides centrifugés en même temps qu'elles et se trouvant à leur contact. A fortiori, si ces corps solides présentent une forme non ramassée et avec des arêtes, comme les filtres susmentionnés, le danger de détérioration, voire d'éclatement des poches, est considérable.

L'invention remédie à ces inconvénients et a notamment pour but de proposer un procédé permettant la centrifugation simultanée et dans un même espace de confinement, de poches et d'au moins un filtre, et qui minimise le risque de détérioration de ces éléments.

Pour un procédé consistant à confiner des ensembles comprenant des poches et au moins un filtre dans un espace de confinement de forme déterminée que l'on fait tourner à grande vitesse autour d'un axe déterminé passant par le milieu dudit espace, ce but est atteint, conformément à l'invention, du fait que l'on place le filtre au milieu de l'espace de confinement de telle sorte que les grandes faces du filtre soient parallèles à l'axe de centrifugation, et du fait que l'on positionne les poches ou ensembles de poches de part et d'autre dudit filtre.

Avantageusement, durant la centrifugation, on maintient positivement le bord inférieur et les bords latéraux du filtre en des positions constantes par rapport à l'espace de confinement.

Avantageusement, on positionne d'abord les poches dans l'espace de confinement, puis on place le filtre entre les poches de telle sorte que chacune de ses grandes faces soient en contact avec une poche.

Dans le cas où l'on traite un ensemble comportant plusieurs filtres, on place ces fitres au milieu de l'espace de confinement, à l'état empilés les uns sur les autres par leurs grandes faces, et on place les poches de part et d'autre dudit empilement.

L'invention concerne aussi un dispositif de centrifugation d'ensembles comprenant des poches à parois souples remplies de liquide et au moins un filtre relié auxdites poches. Ce dispositif comporte un support de centrifugation en forme de récipient ouvert vers le haut ayant un axe central vertical, susceptible d'être entraîné en rotation à une vitesse convenable autour dudit axe, le support matérialisant par ses parois internes ledit espace de confinement.

Ce dispositif est caractérisé, conformément à l'invention, en ce que le support comporte des moyens pour supporter et positionner au moins un filtre et pour constituer avec ce dernier un cloisonnement vertical, de forme générale plane, traversé sensiblement en son plan médian par ledit axe central, et subdivisant le volume intérieur du support en deux compartiments destinés à contenir les poches.

Avantageusement, les moyens de support et de positionnement de filtre comportent des moyens ménageant un logement pour au moins un filtre, ce logement étant délimité vers le bas et latéralement par une surface dont le contour épouse sensiblement la forme de celui du filtre.

Grâce à cette caractéristique, les moyens de logement reçoivent le filtre sensiblement sans jeu parallèlement à son plan, de sorte que ledit contour du logement constitue un moyen maintenant positivement les bords inférieur et latéraux du filtre, dans une position.constante de l'espace de confinement.

Les moyens de support et de positionnement sont, par exemple, constitués par une cloison pleine à faces sensiblement parallèles. Dans ce cas, les moyens de logement sont avantageusement constitués par une simple ouverture qui est ménagée dans ladite paroi à partir de son bord supérieur, et dont le contour épouse sensiblement la forme de celui d'un filtre.

Selon un autre mode de réalisation de l'invention, les moyens de logement comprennent un insert en forme d'étrier, qui est monté de façon amovible dans le support, qui est susceptible de recevoir dans son ouverture au moins un filtre, et dont la surface interne présente un contour épousant sensiblement la forme de celui du filtre.

Il est avantageux que le support de centrifugation présente une section horizontale de forme allongée, et que ledit cloisonnement s'étende selon la petite dimension de cette section horizontale.

Selon un mode de réalisation préféré, chaque compartiment est conformé et dimensionné de manière qu'une seule poche, ou un seul ensemble de poche, disposé dans ce dernier soit en contact avec l'ensemble des parois dudit compartiment, y compris ledit cloisonnement que constituent les moyens de support et de positionnement avec le filtre.

Les avantages de l'invention définie ci-dessus sont multiples :

Elle permet le positionnement des filtres à un endroit et dans une position tels qu'ils ne risquent pas d'endommager les poches.

Elle permet le positionnement des filtres à un endroit où la force centrifuge a la plus basse valeur possible, ce qui limite l'effet de pression sur ces derniers.

Elle permet le maintien des filtres dans un endroit et une position constants, et donc définis à l'avance, par rapport à l'espace de confinement matérialisé par les parois internes du support. Il est ainsi possible de tester préalablement la résistance des filtres aux forces centrifuges avant de les relier à des poches qui seront ultérieurement remplies de sang à traiter.

D'autres avantages et caractéristiques apparaîtront à la lecture de la description qui va suivre de plusieurs modes de réalisation de l'invention, description faite en référence aux dessins annexés dans lesquels :
- la figure 1 est une vue schématique en plan d'un ensemble composé d'une poche et d'un filtre ;
- la figure 2 est une vue en élévation et en coupe verticale selon le plan II-II de la figure 3, d'un support de centrifugation selon un premier mode de réalisation de l'invention ;
- la figure 3 est la vue en plan correspondant à la figure 2 ;
- la figure 4 est une vue en élévation et en coupe verticale selon le plan IV-IV de la figure 3, du support représenté aux figures 2 et 3 ;
- la figure 5 est une vue en élévation et en coupe verticale selon le plan V-V de la figure 6, d'un support de centrifugation selon un deuxième mode de réalisation de l'invention ;
- la figure 6 est la vue en plan correspondant à la figure 5 ;
- la figure 7 est une vue en élévation et en coupe verticale selon le plan VII-VII de la figure 6, du support représenté aux figures 5 et 6 ;
- la figure 8 est une vue en élévation d'un insert destiné à être monté sur le support représenté aux figures 5 à 7;
- la figure 9 représente l'ajustement de l'insert vu en section selon le plan A-A de la figure 8, dans l'échancrure des moyens de paroi du support représenté aux figures 5 à 7 ; et
- la figure 10 est une vue analogue à la figure 9, montrant un insert d'une autre taille adapté à des filtres de plus faibles dimensions.

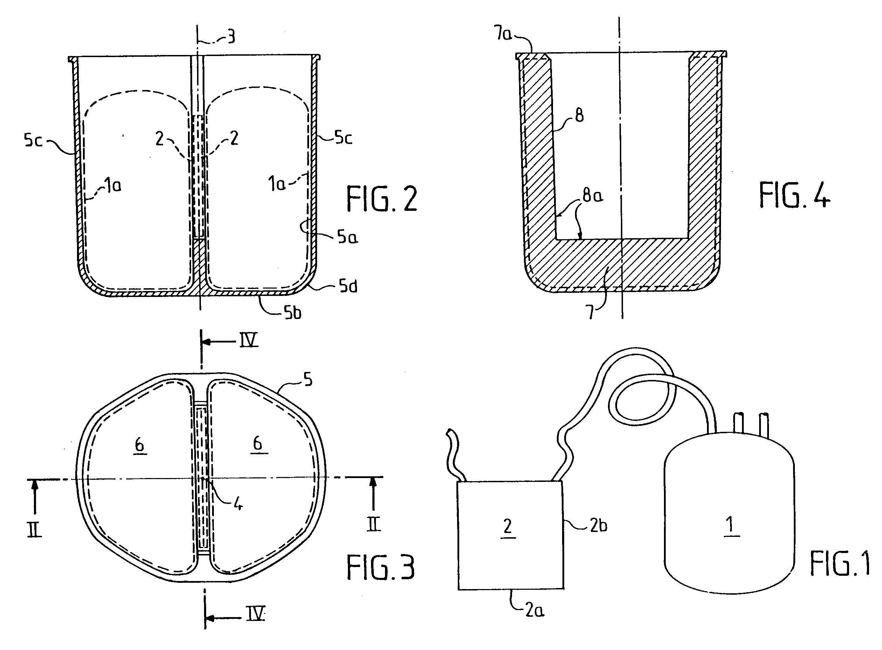

La figure 1 montre un ensemble utilisé dans les procédés de préparation de composés sanguins. Le sang est transfusé dans des poches à parois souples, telles que la poche 1, déjà reliées entre elles par des tubulures souples, et comportant déjà dans le circuit un filtre 2 plat, de forme rectangulaire, qui est composé d'une enveloppe rigide dont le volume intérieur est séparé en deux parties par une paroi filtrante.

L'ensemble formé par les poche 1 et le filtre 2 doit être centrifugé pour séparer les composants du liquide biologique, puis ces composants sont isolés l'un de l'autre par une opération ultérieure de filtrage effectuée au moyen du filtre 2 du circuit.

Comme on peut le voir sur les figure 2 et 3, durant la centrifugation, on positionne les filtres 2 verticalement, alignés et empilés les uns sur les autres par leurs grandes faces de telle sorte que lesdites grandes faces des filtres soient parallèles à l'axe de centrifugation 3 (figure 2), et que ledit axe de centrifugation 3 traverse ledit empilement de filtres 2 sensiblement par le centre 4 de sa section droite (figure 3).

En outre, comme on peut le voir sur les figures 2 et 3, durant la centrifugation, on positionne des ensembles la de poches 1 (ensembles représentés en pointillé sur les figures 2 et 3) de part et d'autre dudit empilement de filtres 2, et on maintient positivement le bord - ou tranche - inférieur 2a et les bords - ou tranches - latéraux 2b des filtres en des positions constantes par rapport à l'espace de confinement qui est matérialisé par la surface interne 5a des parois d'un support - ou portoir - 5 de centrifugation en forme de récipient ouvert vers le haut et à section horizontale de forme générale ovoïde.

L'axe central du portoir 5, c'est-à-dire l'axe reliant les centres de ses sections horizontales, coïncide avec l'axe de centrifugation 3.

La paroi de fond 5b du portoir est reliée aux parois latérales 5c par des courbures douces 5d afin d'épouser la forme naturelle des poches remplies de liquide 1.

Le portoir 5 est, par exemple, réalisé en matière plastique. Il est destiné à être monté dans une nacelle métallique de centrifugation.

En outre, comme on peut le voir sur les figure 2 et 3, durant la centrifugation, l'empilement de filtres 2 est positionné de telle sorte que la grande face libre de chaque filtre extrême de l'empilement soit en contact avec la ou les poche(s) 1 adjacente(s) desdits ensembles de poches 1a.

Deux ensembles de poches la sont placés dans un même portoir 5, chacun à chaque extrémité de l'ovale du portoir 5, de part et d'autre de l'empilement de filtres 2.

Radialement, dans le sens du petit axe du portoir 5, s'étend un cloisonnement vertical, de forme générale plane, traversé sensiblement en son plan médian par l'axe de centrifugation 3. Ce cloisonnement subdivise le volume intérieur du portoir 5 en deux compartiments 6 symétriques l'un de l'autre par rapport au plan médian du cloisonnement, plan qui, sur la figure 3, se confond avec le plan de coupe IV-IV.

Selon un mode de réalisation préféré, chaque compartiment 6 est conformé et dimensionné de manière qu'une seule poche 1 disposée dans ce dernier soit en contact avec l'ensemble des parois dudit compartiment, y compris le cloisonnement.

Ce cloisonnement est constitué par l'empilement de filtres 2 logé dans un logement ménagé dans des moyens de support et de positionnement qui seront décrits en détail ci-dessous.

Selon le mode de réalisation représenté sur les figures 2 à 4, les moyens de support et de positionnement des filtres sont constitués par une simple cloison pleine à faces sensiblement parallèles 7, solidaire du portoir 5, par exemple venue d'une seule pièce avec ce dernier par moulage. Les moyens de logement sont constitués par une ouverture (ou échancrure) rectangulaire 8 ménagée dans la cloison 7 à partir du bord supérieur 7a de cette dernière, cette ouverture recevant l'empilement de filtres 2.

Le contour 8a de l'ouverture 8 épouse sensiblement la forme de celui d'un filtre 2, si bien que les filtres 2 y sont logés pratiquement sans jeu parallèlement à leurs grandes faces.

Il en résulte que le contour 8a de ladite ouverture 8 constitue un moyen de maintien positif des bords inférieur 2a et latéraux 2b des filtres dans une position constante de l'espace de confinement, lorsque les ensembles 1, 2 sont centrifugés et que l'empilement de filtres 2 est serré par les ensembles de poches la situés de part et d'autre de ce dernier.

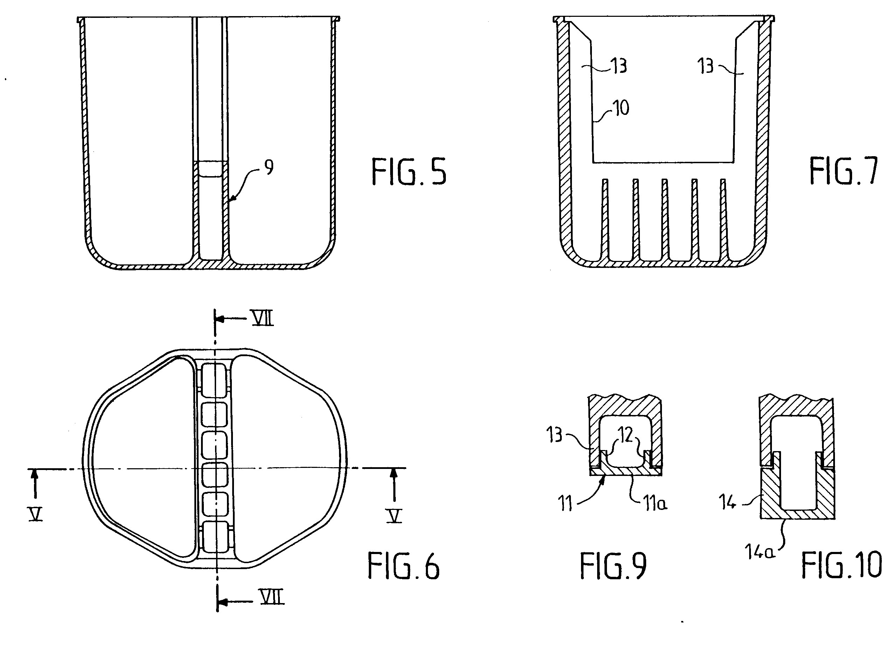

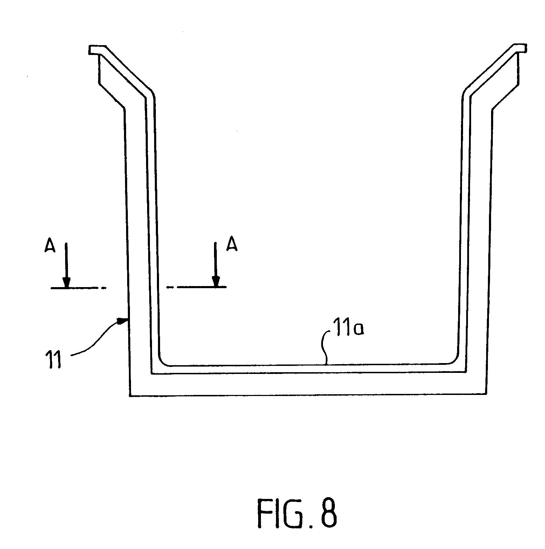

Selon le mode de réalisation représenté sur les figures 5 à 7, les moyens de support et de positionnement des filtres sont constitués par une paroi creuse cloisonnée intérieurement 9 solidaire du portoir 5, paroi qui comporte elle aussi une ouverture 10 de forme rectangulaire ménagée à partir du bord supérieur de ladite cloison 9. Néanmoins ce mode de réalisation diffère du précédent par le fait que les moyens de logement de filtres 2 sont ici matérialisés par un insert amovible 11 en forme générale d'étrier (figure 8) qui est susceptible de se glisser dans l'ouverture 10 jusqu'à buter contre le bord inférieur horizontal 10a de cette dernière. L'insert 11 est maintenu en position vis-à-vis de la paroi 9 grâce à la coopération de nervures périphériques 12 formant organe de guidage s'engageant, avec jeu de glissement, dans une glissière formée par des nervures 13 de la paroi 9, nervures s'étendant vers l'intérieur de l'ouverture 10 et sur la longueur des bords latéraux verticaux de cette dernière. Les nervures 12 et 13 constituent ainsi des moyens de montage amovible de l'insert 11 sur la cloison 9.

La surface interne lla de l'étrier 11 est conformée et dimensionnée de manière à épouser sensiblement la forme du pourtour rectangulaire d'un filtre 2.

Le mode de réalisation du portoir avec insert présente, par rapport à celui du portoir sans insert représenté sur les figures 2 à 4, l'avantage qu'un même portoir peut servir successivement pour des filtres de différentes formes et/ou tailles : il suffit pour cela de changer d'insert. Sur la figure 10, on a représenté la section droite d'un insert 14 dont l'ouverture est de plus petites dimensions. La surface interne 14a de l'insert 14 épouse le contour d'un filtre de dimensions plus faibles que celui qui est adapté à l'insert 11 de la figure 9.

Les portoirs qui viennent d'être décrits sont avantageusement utilisés de la manière suivante :

Les filtres 2 sont placés verticalement dans l'ouverture 8 de la cloison 7 ou dans l'insert 11 monté dans la cloison 9, après que les poches souples 1 ont été placées dans leurs logements 6 respectifs. Il n'y a donc aucune paroi séparant les filtres 2 des poches 1. Les filtres 2 sont maintenus dans cette position par la pression que les poches 1 exercent sur eux. La force centrifuge augmentant pendant l'opération de centrifugation, les filtres 2 restent dans leur position initiale.

Ainsi, il n'y a aucune perte de place puisqu'il n'y a pas de paroi séparant les filtres 2 des poches 1. On obtient ainsi un portoir dont les dimensions sont les plus réduites possibles.

Du fait que le logement (ouverture 8 de la cloison 7, ou l'étrier 11) destiné à recevoir les filtres 2, présente, dans sa périphérie, la forme du filtre 2, ce dernier, lors de la centrifugation, repose sur la plus grande surface possible, réduisant ainsi au minimum l'effort supporté par le filtre et développé par la force centrifuge.

Cet effort est encore plus minimisé par le fait que la profondeur de ce logement (épaisseur de l'ouverture 8 ou de l'étrier 11) est choisie la plus faible possible.

Ainsi, grâce à l'invention, la pression exercée par les poches 1 sur les filtres 2 et la poussée de la force centrifuge sur les filtres 2 eux-mêmes, sont minimales.

Enfin, la situation de ce logement pour filtre, qui est de forme générale plane, reste compatible avec la forme que peuvent prendre les poches remplies de liquide 1, sous l'effet de la force centrifuge.

Pour l'utilisateur, la forme même du portoir, avec son ouverture 8 ou 10, évite toute équivoque quant à l'endroit où doivent être placés les filtres 2. Il en résulte une réduction importante du risque de casse des filtres ou de percement des poches par les filtres qui se traduisent par un risque majeur de contamination de l'environnement.

## Revendications

1. Procédé de traitement par centrifugation d'un liquide conditionné dans des poches à parois souples (1) reliées, par l'intermédiaire d'une tubulure souple, à au moins un filtre (2) de forme sensiblement plate, selon lequel on place les poches (1) avec le filtre (2) dans un espace de confinement de forme déterminée (5, 5a) que l'on fait tourner à grande vitesse autour d'un axe déterminé (3) passant par le milieu dudit espace, caractérisé en ce qu'on place le filtre (2) au milieu de l'espace de confinement (5, 5a) de telle sorte que les grandes faces du filtre (2) soient parallèles à l'axe de centrifugation (3), et on place les poches (1) de part et d'autre dudit filtre.

2. Procédé selon la revendication 1, caractérisé en ce que, durant la centrifugation, on maintient positivement le bord inférieur (2a) et les bords latéraux (2b) du filtre (2) en position constante par rapport à l'espace de confinement (5, 5a).

3. Procédé selon l'une des revendications 1 et 2, caractérisé en ce qu'on place d'abord les poches (1) dans ledit espace de confinement (5, 5a) puis on positionne le filtre (2) entre les poches (1) de sorte qu'il soit en contact avec une poche (1) par chacune de ses grande faces.

4. Procédé selon l'une des revendications 1 à 3 et selon lequel on traite plusieurs filtres (2), caractérisé en ce qu'on dispose les filtres (2) au milieu de l'espace de confinement (5, 5a), à l'état empilés les uns sur les autres par leurs grandes faces.

5. Dispositif pour la mise en oeuvre du procédé selon l'une des revendications précédentes, et comportant un support de centrifugation en forme de récipient ouvert vers le haut (5) ayant un axe central vertical (3), susceptible d'être entraîné en rotation à grande vitesse autour dudit axe (3), le support (5) comportant des moyens (7; 9,11; 9,14) pour supporter et positionner au moins un filtre (2), caractérisé en ce que le filtre constitue avec ledit support un cloisonnement vertical, de forme générale plane, traversé sensiblement en son plan médian par ledit axe central (3), et subdivisant le volume intérieur du support en deux compartiments (6) destinés à contenir les poches (1).

6. Dispositif selon la revendication 5, caractérisé en ce que les moyens de support et de positionnement comportent des moyens de logement (8, 8a; 11,11a; 14, 14a) pour au moins un filtre (2), délimités vers le bas et latéralement par une surface (8a; 11a; 14a) dont le contour épouse sensiblement la forme de celui d'un filtre (2).

7. Dispositif selon l'une des revendications 5 et 6, caractérisé en ce que lesdits moyens de support et de positionnement comprennent une cloison pleine (7) à faces sensiblement parallèles (7), solidaire dudit support (5), et en ce que les moyens de logement sont constitués par une ouverture (8) ménagée dans ladite paroi (7), de préférence à partir du bord supérieur (7a) de cette dernière, et dont le contour (8a) épouse sensiblement la forme de celui d'un filtre (2).

8. Dispositif selon l'une des revendications 5 et 6, caractérisé en ce que lesdits moyens de logement comprennent un insert en forme générale d'étrier (11, 14), qui est susceptible de recevoir au moins un filtre (2), et dont la surface interne (11a; 14a) présente un contour épousant sensiblement la forme de celui d'un filtre (2), et en ce que des moyens (12, 13) sont prévus pour le montage amovible de l'insert (11; 14) dans le support (5).

9. Dispositif selon l'une des revendications 5 à 8, caractérisé en ce que ledit support (5) présente, en section horizontale, une forme allongée, de préférence une forme ovoïde, et en ce que ledit cloisonnement (2, 7; 2, 9,11; 2, 9,14) s'étend selon la petite dimension de cette section horizontale.

10. Dispositif selon l'une des revendications 5 à 9, caractérisé en ce que chaque compartiment (6) est conformé et dimensionné de manière qu'une seule poche (1) ou ensemble de poches disposé dans ce dernier soit en contact avec l'ensemble des parois (5a, 2 7; 9, 11; 9, 14) dudit compartiment (6).

## Claims

1. Method for treatment by centrifuging of a liquid packed in bags with flexible walls (1) connected by means of a flexible tube to at least one filter (2) of a substantially flat shape, according to which the bags (1) are placed with the filter (2) in a containment space of a specific shape (5, 5a) which is caused to rotate at high speed about a specific axis (3) passing through the centre of said space, characterised in that the filter (2) is placed in the centre of the containment space (5, 5a) such that the large faces of the filter (2) are parallel with the centrifugation axis (3), and the bags (1) are placed on either side of said filter.

2. Method according to claim 1, characterised in that, during centrifuging, the lower edge (2a) and the lateral edges (2b) of the filter are kept positively in a position constant with respect to the containment space (5, 5a).

3. Method according to one of claims 1 and 2, characterised in that firstly the bags (1) are placed in said containment space (5, 5a), then the filter (2) is positioned between the bags (1) such that it is in contact with a bag (1) with each of its large faces.

4. Method according to one of claims 1 to 3 and according to which a plurality of filters (2) are treated, characterised in that the filters (2) are disposed in the centre of the containment space (5, 5a) in a condition stacked on one another by their large faces.

5. Device for putting into effect the method according to one of the preceding claims, and comprising a centrifuging support in the form of an upwardly open container (5) having a vertical central axis (3), capable of being rotated at high speed about said axis (3), the support(5) comprising means (7; 9,11; 9,14) of supporting and positioning at least one filter (2), characterised in that the filter constitutes with said support a vertical partition of a generally flat shape, through the median plane of which the said central axis (3) substantially passes, subdividing the interior volume of the support into two compartments (6) intended to contain the bags (1).

6. Device according to claim 5, characterised in that the supporting and positioning means comprise housing means (8,8a; 11,11a; 14,14a) for at least one filter (2), defined towards the foot and laterally by a surface (8a; 11a; 14a) whose contour substantially conforms to the shape of a filter (2).

7. Device according to one of claims 5 and 6, characterised in that the said supporting and positioning means comprise a solid partition (7) with substantially parallel faces (7), integral with said support (5), and in that the housing means are constituted by an aperture (8) produced in said partition (7), preferably from the upper edge (7a) of the latter, and whose contour (8a) substantially conforms to the shape of a filter (2).

8. Device according to one of claims 5 and 6, characterised in that said housing means comprise an insert in the general shape of a clamp (11, 14) which is capable of accommodating at least one filter (2), and the internal surface (11a; 14a) of which has a contour substantially conforming to the shape of a filter (2), and in that means (12, 13) are provided for movably mounting the insert (11; 14) in the support (5).

9. Device according to one of claims 5 to 8, characterised in that said support (5) has an elongate shape, preferably an ovoid shape, in horizontal cross-section, and in that the said partition (2,7; 2,9,11; 2,9,14) extends in accordance with the small dimension of this horizontal cross-section.

10. Device according to one of claims 5 to 9, characterised in that each compartment (6) is shaped and dimensioned such that one single bag (1) or assembly of bags disposed inside the latter is in contact with all the partitions (5a,2,7; 9,11; 9,14)of said compartment (6)

## Patentansprüche

1. Verfahren zur Zentrifugalbehandlung einer Flüssigkeit, welche in Taschen mit flexiblen Wänden (1) eingefüllt ist, die mit wenigstens einem Filter (2) von im wesentlichen Plattengestalt unter Zwischensetzen eines elastischen Stutzen verbunden sind, mit den Schritten: Setzen der Taschen (1) mit dem Filter (2) in einen eingeschlossenen Raum bestimmter Form (5, 5a), den man mit hoher Geschwindigkeit um eine bestimmte Achse (3), welche durch die Mitte des Raums läuft, drehen läßt, dadurch gekennzeichnet, daß man den Filter (2) derart in die Mitte des eingschlossenen Raums (5, 5a) setzt, daß die Hauptflächen des Filters (2) parallel zur Zentrifugalachse (3) sind, und daß man die Taschen (1) beiderseits des Filters anordnet.

2. Verfahren nach Anspruch 1, dadurch gekennzeichnet, daß während der Zentrifugalbehandlung der untere Rand (2a) und die Seitenränder (2b) des Filters (2) in konstanter Position bezüglich des eingeschlossenen Raums (5, 5a) festgehalten werden.

3. Verfahren nach einem der Ansprüche 1 und 2, dadurch gekennzeichnet, daß man zunächst die Taschen (1) in den eingeschlossenen Raum (5, 5a) setzt, und dann den Filter (2) derart zwischen die Taschen (1) setzt, daß er in Kontakt mit einer Tasche (1) über jede seiner Hauptflächen steht.

4. Verfahren nach einem der Ansprüche 1 bis 3, wobei man mehrere Filter (2) anbringt, dadurch gekennzeichnet, daß man die Filter (2) in die Mitte des eingeschlossenen Raums (5, 5a) setzt, und zwar in dem Zustand, daß sie übereinander über ihren Hauptflächen aufeinandergesetzt sind.

5. Vorrichtung zur Durchführung des Verfahrens nach einem der vorhergehenden Ansprüche mit einer Zentrifugalbehandlungshalterung in Form eines nach oben (5) offenen Behälters, der eine zentrale vertikale Achse (3) aufweist, der mit großer Geschwindigkeit um die Achse (3) drehbar ist, wobei die Halterung (5) Mittel (7; 9, 11; 9, 14) zum Haltern und Positionieren zumindest eines Filters (2) aufweist, dadurch gekennzeichnet, daß der Filter mit der Halterung eine vertikale Unterteilung von im wesentlichen ebener Gestalt bildet, die im wesentlichen in ihrer Mittelebene von der zentralen Achse (3) durchsetzt ist und das Innenvolumen der Vorrichtung in zwei Abteile (6) teilt, welche zur Aufbewahrung der Taschen (1) bestimmt sind.

6. Vorrichtung nach Anspruch 5, dadurch gekennzeichnet, daß die Mittel zur Halterung und Positionierung Unterbringungsmittel (8, 8a; 11, 11a; 14, 14a) für zumindest einen Filter (2) aufweisen, welche nach unten und seitlich durch eine Fläche (8a; 11a; 14a) begrenzt sind, deren Kontur im wesentlichen die Form dieses einen Filters (2) annimmt.

7. Vorrichtung nach einem der Ansprüche 5 und 6, dadurch gekennzeichnet, daß die Mittel zum Haltern und Positionieren eine massive Wand (7) mit im wesentlichen parallelen Flächen (7), die einstückig mit der Vorrichtung (5) ist, aufweist und daß die Unterbringungsmittel aus einer Öffnung (8) gebildet sind, die in der Wand (7) untergebracht ist, und zwar vorzugsweise ausgehend von der oberen Kante (7a) der letzteren, und deren Kontur (8a) im wesentlichen die Form dieses einen Filters (2) annimmt.

8. Vorrichtung nach einem der Ansprüche 5 und 6, dadurch gekennzeichnet, daß die Unterbringungsmittel einen Einsatz mit der allgemeinen Form eines Bügels (11, 14) aufweisen, der zumindest einen Filter (2) aufnehmen kann und dessen innere Oberfläche (11a; 14a) eine Kontur aufweist, welche im wesentlichen die Form dieses einen Filters (2) annimmt, und daß die Mittel (12, 13) zur abnehmbaren Montage des Einsatzes (11; 14) in der Halterung (5) vorgesehen sind.

9. Vorrichtung nach einem der Ansprüche 5 bis 8, dadurch gekennzeichnet, daß die Halterung (5) im horizontalen Querschnitt eine längliche Form aufweist, und zwar vorzugsweise eine ovale Form, und daß die Wand (2, 7; 2, 9, 11; 2, 9, 14) entlang der kleinen Dimension dieses horizontalen Querschnitts verläuft.

10. Vorrichtung nach einem der Ansprüche 5 bis 9, dadurch gekennzeichnet, daß jedes Abteil (6) derart gestaltet und dimensioniert ist, daß eine einzelne Tasche (1) oder eine Ansammlung von Taschen, welche darin angeordnet ist, in Kontakt mit der Anordnung der Wände (5a, 2, 7; 9, 11; 9, 14) des Abteils (6) ist.
